# FASCICULE DE BREVET EUROPEEN

(11) **EP 1 579 924 B1**
(45) Date de publication et mention de la délivrance du brevet: **14.12.2011**
(21) Numéro de dépôt: 05300195.4
(22) Date de dépôt: 18.03.2005
(51) Int. Cl.: B05B 11/00

(54) **Ensemble de conditionnement et de distribution d'un produit fluide**
Verpackungs- und Spendeeinheit für ein flüssiges Produkt
Packaging and distribution assembly for a fluid product

(30) Priorité: 24.03.2004 FR 0403024
(43) Date de publication de la demande: 28.09.2005
(73) Titulaire: L'Oréal, 75008 Paris (FR)
(72) Inventeur: Ramet, Marc, F-92600 Asnières (FR)
(74) Mandataire: Tanty, François

(56) Documents cités:
- FR-A- 2 430 269
- FR-A- 2 705 039
- US-A- 3 718 165
- US-A- 5 894 958

## Description

La présente invention a pour objet un ensemble de conditionnement et de distribution d'un produit, notamment cosmétique, y compris de soin.

Par « produit cosmétique », on entend au sens de la présente invention un produit tel que défini dans la Directive 93/35/CEE du 14 juin 1993 modifiant la Directive 76/768/CEE.

On connaît par la demande de brevet FR 2 773 443 un atomiseur rechargeable, comportant un corps définissant une chambre pour le stockage d'un produit. L'atomiseur comporte en outre un piston coulissant dans le corps et pouvant être enfoncé pour réduire le volume de la chambre et distribuer du produit. Selon la course d'enfoncement du piston, une certaine quantité de produit est distribuée, la chambre étant entièrement vidée lorsque le piston est enfoncé sur une course complète. L'atomiseur ne permet pas de distribuer facilement et avec précision une dose de produit correspondant à une fraction du volume maximal de la chambre.

La demande de brevet FR 2 705 039 montre les caractéristiques des préambules des revendications 1 et 35 et décrit un dispositif de distribution comportant un récipient, contenant une réserve de produit, muni d'une première pompe, et un flacon rechargeable muni d'une deuxième pompe. Le flacon peut être rechargé en produit par l'intermédiaire de la première pompe. Pour distribuer du produit contenu dans le flacon, l'utilisateur sépare le flacon du récipient puis actionne la deuxième pompe.

La demande de brevet FR 2 813 291 décrit un système de remplissage d'un flacon secondaire à partir d'un flacon principal. Le flacon principal est équipé de canules permettant de mettre les flacons principal et secondaire en communication fluidique. Lorsque le flacon secondaire est équipé d'une pompe, cette dernière doit être retirée avant le raccordement des flacons principal et secondaire pour le rechargement.

La demande de brevet FR 2 802 447 décrit un système vaporisateur rechargeable, comportant un réservoir et un vaporisateur équipé d'une pompe. Le réservoir et le vaporisateur sont pourvus de conduits pouvant être raccordés temporairement afin de permettre le rechargement du vaporisateur. Le conduit s'étendant dans le vaporisateur est ouvert à son extrémité supérieure pour permettre l'écoulement de produit directement dans la réserve du vaporisateur.

La demande de brevet FR 2 556 091 décrit un dispositif rechargeable amovible comportant un corps et un piston coulissant dans le corps. Ce dispositif est dépourvu de pompe.

La présente invention vise à fournir un ensemble de conditionnement et de distribution qui présente une structure relativement simple et permette de distribuer de manière relativement précise le produit.

L'invention a ainsi pour objet un ensemble de conditionnement et de distribution d'un produit, comportant :
- un récipient pour contenir une réserve de produit,
- une unité rechargeable agencée pour être disposée de façon amovible sur le récipient, l'unité rechargeable comportant :
   - une chambre de stockage pour contenir du produit et capable d'être mise en communication fluidique avec le récipient pour son remplissage lorsque l'unité rechargeable est disposée sur le récipient,
   - une pompe capable de prélever du produit dans le récipient lorsque l'unité rechargeable est disposée dessus et de prélever du produit dans la chambre de stockage lorsque l'unité rechargeable est séparée du récipient, la pompe comportant une chambre de pompage distincte de la chambre de stockage, c'est-à-dire non confondue avec celle-ci.

Grâce à l'invention, lorsque l'unité rechargeable est utilisée séparément du récipient, il est possible de distribuer de manière relativement précise une ou plusieurs doses de produit correspondant chacune à une fraction seulement du volume maximal de la chambre de stockage. La quantité de produit correspondant à une dose est déterminée par le volume maximal de la chambre de pompage, ce volume maximal de la chambre de pompage étant inférieur au volume maximal de la chambre de stockage.

La pompe peut servir également à prélever du produit dans la réserve de produit du récipient.

Lorsque le récipient comporte un orifice de sortie apte à être mis en communication fluidique avec la chambre de stockage de l'unité rechargeable pour son remplissage, le récipient peut être dépourvu de pompe capable d'alimenter en produit ledit orifice de sortie. Le récipient peut ainsi présenter une structure relativement simple.

Ainsi, l'ensemble selon l'invention peut comporter une pompe unique, celle de l'unité rechargeable, ce qui permet de réduire les coûts.

Avantageusement, l'unité rechargeable est agencée pour permettre de générer une dépression dans la chambre de stockage, au moins lorsque la chambre de stockage est en communication fluidique avec le récipient, de manière à permettre le remplissage de la chambre de stockage. Cette dépression peut être générée au moyen de la pompe ou autrement.

Dans un exemple de mise en oeuvre de l'invention, la chambre de pompage est isolée de la chambre de stockage au moins lors de la distribution de produit par la pompe.

Dans un exemple de mise en oeuvre de l'invention, la pompe comporte un orifice d'aspiration, lequel est apte à être disposé à proximité d'un orifice de sortie du récipient, par exemple à l'aplomb de cet orifice de sortie, lorsque l'unité rechargeable est disposée sur le récipient.

Ainsi, le produit quittant le récipient par l'orifice de sortie peut s'écouler vers l'orifice d'aspiration de la pompe suivant un trajet relativement court, ce qui limite les pertes de charge.

Dans un exemple de mise en oeuvre de l'invention, la pompe est une pompe sans reprise d'air. Cette pompe peut être avec ou sans précompression.

Le récipient peut comporter un support agencé pour recevoir l'unité rechargeable, ledit support étant par exemple immobile par rapport au reste du récipient. Le support peut être une pièce rapportée ou être réalisé de façon monolithique avec le récipient.

L'unité rechargeable et le récipient peuvent comporter des parties de fixation respectives aptes à coopérer de manière amovible, notamment par encliquetage, vissage par une fixation de type baïonnette, par friction ou autrement encore. Cela peut permettre de solidariser l'unité rechargeable du récipient pendant le remplissage de la chambre de stockage et/ou lorsque la pompe de l'unité rechargeable est utilisée pour prélever du produit dans le récipient.

Avantageusement, l'un au moins de l'unité rechargeable et du récipient comporte des reliefs aptes à coopérer avec l'autre de l'unité rechargeable et du récipient pour isoler de manière étanche l'orifice d'aspiration de la pompe de l'extérieur.

Le récipient peut comporter un passage de reprise d'air permettant une reprise d'air lorsque la pompe de l'unité rechargeable est utilisée pour prélever du produit dans le récipient.

En variante, le récipient est sans reprise d'air et comporte notamment un piston ou une poche en contact avec le produit. Le récipient peut par exemple comporter un piston qui se déplace dans celui-ci en réponse au départ du produit.

Le produit peut encore par exemple être contenu dans une poche souple.

Dans un exemple de mise en oeuvre de l'invention, la chambre de stockage présente un volume sensiblement constant.

L'unité rechargeable peut comporter un premier orifice apte à être mis en communication fluidique avec la pompe et le récipient pour permettre à la pompe de prélever du produit dans le récipient lorsque l'unité rechargeable est disposée sur le récipient, et un deuxième orifice distinct du premier, apte à être mis en communication fluidique avec le récipient pour le remplissage de la chambre de stockage.

Le deuxième orifice peut être réalisé sur une paroi de fond de l'unité rechargeable.

L'unité rechargeable peut comporter un clapet capable d'isoler l'orifice d'aspiration de la chambre de stockage lorsque l'unité rechargeable est disposée sur le récipient, ce dernier étant tête en haut, et de permettre une communication fluidique entre ledit orifice d'aspiration et la chambre de stockage lorsque l'unité rechargeable est disposée sur le récipient, l'ensemble étant tête en bas.

L'unité rechargeable peut comporter une paroi, notamment une paroi de fond, agencée pour former avec une paroi du récipient, lorsque l'unité rechargeable est disposée sur le récipient, un passage à l'extérieur de la chambre de stockage. Ce passage peut mettre en communication fluidique l'orifice d'aspiration de la pompe avec la chambre de stockage. Ce passage peut être obturé par le clapet précité.

Le récipient peut comporter un premier orifice de sortie apte à être mis en communication fluidique avec le premier orifice de l'unité rechargeable lorsque l'unité rechargeable est disposée sur le récipient, et un deuxième orifice de sortie apte à être mis en communication fluidique avec le deuxième orifice de l'unité rechargeable lorsque l'unité rechargeable est disposée sur le récipient.

Le récipient peut comporter un clapet capable d'obturer le premier orifice de sortie du récipient lorsque l'ensemble est tête en bas et de le libérer lorsque le récipient est tête en haut.

L'unité rechargeable peut comporter un corps sur lequel sont réalisés les premier et deuxième orifices et un bouchon agencé pour être fixé de manière amovible sur ledit corps lorsque l'unité rechargeable est séparée du récipient, afm d'obturer lesdits premier et deuxième orifices.

Le bouchon peut former avec le corps de l'unité rechargeable, lorsqu'ils sont assemblés, un passage, à l'extérieur de la chambre de stockage, entre les premier et deuxième orifices, permettant de mettre en communication fluidique la pompe avec la chambre de stockage.

Le bouchon peut encore définir avec le corps de l'unité rechargeable, lorsqu'ils sont assemblés, un passage de reprise d'air permettant une reprise d'air lorsque la pompe est utilisée pour prélever du produit dans la chambre de stockage.

Dans un autre exemple de mise en oeuvre de l'invention, l'unité rechargeable comporte un corps et un piston mobile par rapport au corps et défmissant avec celui-ci une chambre de stockage de volume variable.

De préférence, lorsque l'unité rechargeable est disposée sur le récipient de manière à ce que la pompe puisse prélever du produit dans celui-ci, la pompe communiquant par exemple avec un tube plongeur du récipient, le volume de la chambre de stockage est minimal, étant par exemple sensiblement nul.

Dans un exemple de mise en oeuvre de l'invention, la chambre de stockage est formée sous le piston, lorsque l'unité rechargeable est observée tête en haut.

L'unité rechargeable peut comporter un ensemble mobile par rapport audit corps, l'ensemble mobile comprenant le piston.

Avantageusement, l'ensemble mobile comporte un fourreau extérieur coulissant sur le corps.

L'ensemble mobile peut également comporter une pompe, laquelle peut comporter un conduit, le piston étant fixé autour dudit conduit. Le piston présente avantageusement une face inférieure en retrait de l'extrémité inférieure du conduit, ce dernier comportant une portion se prolongeant vers le bas, sous le piston.

Ainsi, lorsque l'orifice d'aspiration de la pompe est réalisé à l'extrémité inférieure de la portion de conduit précitée, cet orifice d'aspiration se situe à une certaine distance de la face inférieure du piston, ce qui lui permet de plonger dans le produit contenu dans la chambre de stockage, y compris en cas de présence d'une couche d'air dans la partie supérieure de la chambre de stockage.

Le corps de l'unité rechargeable peut comporter un orifice permettant de mettre en communication fluidique la chambre de stockage avec le récipient.

L'unité rechargeable peut comporter un bouchon apte à être fixé de manière amovible, notamment par vissage ou encliquetage, sur le corps pour obturer ledit orifice lorsque l'unité rechargeable est séparée du récipient.

Le récipient peut contenir un produit cosmétique, y compris de soin, notamment un parfum. Le produit peut encore être une crème, par exemple.

L'invention a encore pour objet une unité rechargeable agencée pour être disposée de façon amovible sur un récipient, l'unité rechargeable comportant :
- une chambre de stockage pour contenir du produit et capable d'être mise en communication fluidique avec le récipient pour son remplissage lorsque l'unité rechargeable est disposée sur le récipient,
- une pompe capable de prélever du produit dans le récipient lorsque l'unité rechargeable est disposée dessus et de prélever du produit dans la chambre de stockage lorsque l'unité rechargeable est séparée du récipient, la pompe comportant une chambre de pompage distincte de la chambre de stockage.

L'unité rechargeable peut être agencée pour permettre de générer une dépression dans la chambre de stockage, au moins lorsque la chambre de stockage est en communication fluidique avec le récipient, de manière à permettre le remplissage de la chambre de stockage sous l'effet de la dépression ainsi créée.

Le volume de la chambre de pompage est de préférence inférieur au volume maximal de la chambre de stockage.

Dans un exemple de mise en oeuvre de l'invention, la chambre de stockage présente un volume sensiblement constant.

L'unité rechargeable peut comporter un premier orifice apte à être mis en communication fluidique avec la pompe et le récipient pour permettre à la pompe de prélever du produit dans le récipient lorsque l'unité rechargeable est disposée sur le récipient, et un deuxième orifice distinct du premier, apte à être mis en communication fluidique avec le récipient pour le remplissage de la chambre de stockage.

En variante, l'unité rechargeable comporte un corps et un piston mobile par rapport au corps et définissant avec celui-ci une chambre de stockage de volume variable.

Dans un exemple de mise en oeuvre de l'invention, la chambre de stockage est située sous le piston, lorsque l'unité rechargeable est observée tête en haut.

La pompe peut comporter un conduit, le piston étant fixé autour dudit conduit. Le piston présente avantageusement une face inférieure en retrait de l'extrémité inférieure du conduit, ce dernier comportant une portion se prolongeant vers le bas, sous le piston.

L'invention a encore pour objet, selon un autre de ses aspects, un procédé de distribution d'un produit, comportant les étapes suivantes :
- fournir un ensemble de conditionnement et de distribution, comportant :
   - un récipient pour contenir une réserve de produit,
   - une unité rechargeable agencée pour être disposée de façon amovible sur le récipient et comportant une chambre de stockage et une pompe,
- prélever du produit à l'aide de l'unité rechargeable soit dans le récipient lorsque l'unité rechargeable est disposée sur le récipient, soit dans la chambre de stockage de l'unité rechargeable lorsque l'unité rechargeable est séparée du récipient,
- distribuer une dose de produit prélevée à l'aide de l'unité rechargeable en actionnant la pompe sur une course complète, la dose délivrée correspondant à une fraction seulement du volume maximal de la chambre de stockage, notamment à moins du dixième.

La pompe comportant un orifice d'aspiration et le récipient un orifice de sortie, le procédé peut comporter les étapes suivantes :
- mettre l'orifice d'aspiration en communication avec l'orifice de sortie,
- prélever du produit dans le récipient.

Le procédé peut comporter les étapes suivantes :
- disposer l'unité rechargeable sur le récipient, l'ensemble étant tête en bas,
- actionner la pompe à plusieurs reprises sur une course complète, pour créer une dépression dans la chambre de stockage et permettre le remplissage en produit de celle-ci, sans que le produit ne soit distribué entre deux courses complètes.

En variante, l'unité rechargeable comportant un corps et un piston coulissant dans le corps, le procédé comporte les étapes suivantes :
- disposer l'unité rechargeable sur le récipient,
- déplacer le piston relativement au corps afin d'augmenter le volume de la chambre de stockage, le piston étant lié fixement à la pompe lors de ce déplacement.

La présente invention pourra être mieux comprise à la lecture de la description détaillée qui va suivre, d'exemples de mise en oeuvre non limitatifs de celle-ci, et à l'examen du dessin annexé, sur lequel :
- la figure 1 représente, schématiquement et partiellement, un ensemble de conditionnement et de distribution conforme à l'invention,
- la figure 2 représente, schématiquement et partiellement, en coupe axiale, l'ensemble de la figure 1,
- la figure 3 représente, schématiquement et partiellement, en coupe axiale, la pompe de l'unité rechargeable de l'ensemble des figures 1 et 2,
- la figure 4 est une vue schématique et partielle, en coupe axiale, de l'ensemble de la figure 2, lors du remplissage de l'unité rechargeable,
- la figure 5 est une vue schématique et partielle, en coupe axiale, de l'unité rechargeable séparée du récipient,
- la figure 6 représente, schématiquement et partiellement, en coupe axiale, un ensemble de conditionnement et de distribution conforme à un autre exemple de mise en oeuvre de l'invention,
- la figure 7 représente, schématiquement et partiellement, l'ensemble de la figure 6 en fin de remplissage,
- la figure 8 est une vue schématique et partielle, en coupe axiale, de l'unité rechargeable de l'ensemble des figures 6 et 7, séparée du récipient,
- la figure 9 représente, schématiquement et partiellement, un ensemble de conditionnement et de distribution conforme à un autre exemple de mise en oeuvre de l'invention, et
- la figure 10 représente, schématiquement et partiellement, en coupe axiale, un ensemble de conditionnement et de distribution conforme à un autre exemple de mise en oeuvre de l'invention.

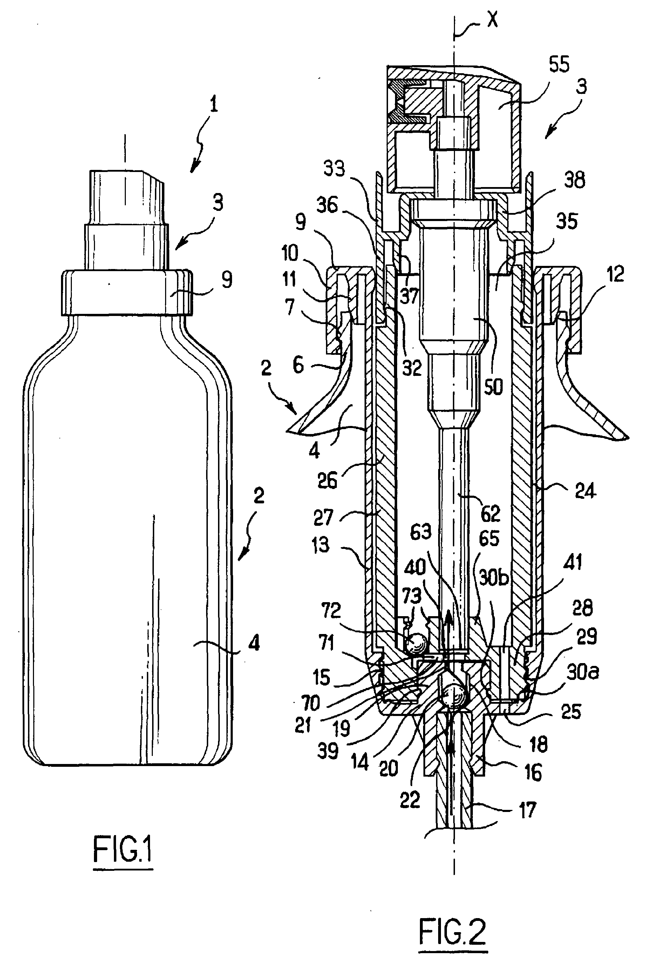

On a représenté sur la figure 1 un ensemble de conditionnement et de distribution 1 conforme à l'invention, comportant un récipient 2 pour contenir une réserve de produit et une unité rechargeable 3 agencée pour être disposée de façon amovible sur le récipient 2.

Dans l'exemple considéré, le produit est un produit cosmétique, notamment un parfum ou un autre produit faiblement visqueux, contenant par exemple un solvant alcoolique. En variante, le produit peut être une crème, par exemple une crème de soin, un lait ou un autre produit à appliquer sur une partie du corps ou du visage, y compris les cheveux.

Le récipient 2 comporte un corps de récipient 4 pourvu à son extrémité supérieure d'un col 6. Ce dernier comporte un bourrelet 7 permettant la fixation d'un support 9 destiné à recevoir l'unité rechargeable 3.

Le support 9 qui peut être réalisé d'une façon monolithique peut comporter, comme on le voit sur la figure 2, une jupe de montage 10 coopérant avec le bourrelet 7 du col 6. Dans l'exemple considéré, la jupe de montage 10 est encliquetée sur le col 6 mais, en variante, elle pourrait être fixée autrement, par exemple par vissage ou sertissage.

L'un au moins du col 6 ou du support 9 peut comporter, le cas échéant, un moyen d'anti-rotation, par exemple un relief (non représenté), s'opposant à la rotation du support 9 par rapport au récipient 2.

Le support 9 est muni d'une lèvre d'étanchéité 11 venant s'appliquer contre la surface intérieure du col 6, pour empêcher la sortie de produit, tout en ménageant un passage de reprise d'air 12 entre l'intérieur du récipient 2 et l'extérieur.

Le support 9 comporte en outre une paroi sensiblement cylindrique 13, d'axe X. Cette paroi 13 s'étend sensiblement sur toute la hauteur du support 9 à l'intérieur du corps 4 et se raccorde inférieurement à une paroi de fond 14 par l'intermédiaire d'une partie filetée intérieurement 15.

La paroi 13 peut présenter une section transversale circulaire, elliptique ou autre.

Les parois 13 et 14 définissent à l'intérieur du support 9 un logement 24 pour recevoir l'unité rechargeable 3.

La paroi de fond 14 porte sur sa face inférieure un embout 16 servant à la fixation d'un tube plongeur 17 et sur sa face supérieure une partie 19 de raccordement étanche avec l'unité rechargeable 3.

Cette partie de raccordement 19 définit un logement 18 communiquant d'une part inférieurement avec le tube plongeur 17 par une ouverture 22 et d'autre part supérieurement avec l'extérieur du récipient 2 au travers d'un premier orifice de sortie 21.

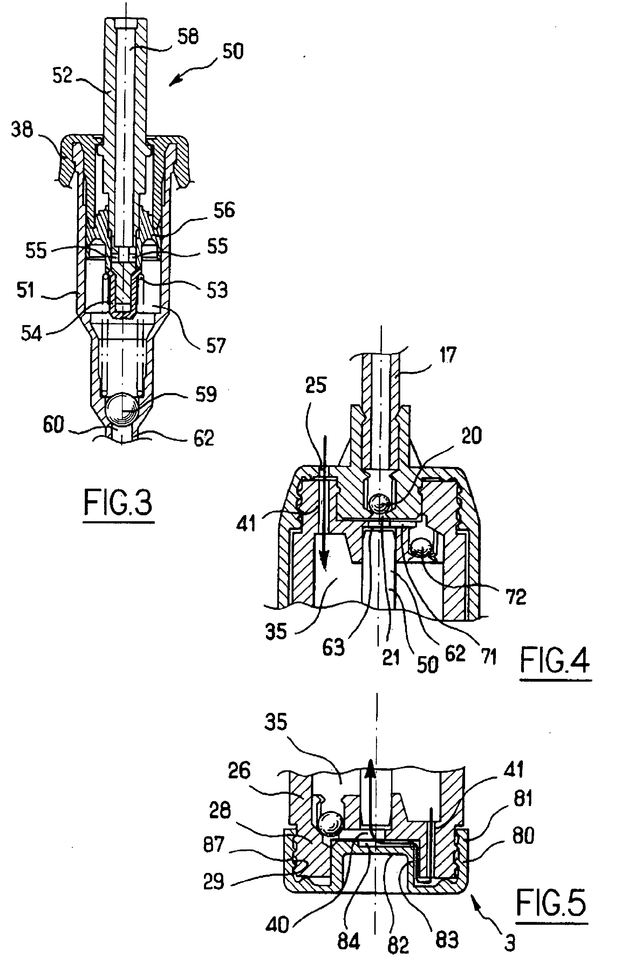

Le logement 18 reçoit une bille 20 de manière à former un clapet capable d'obturer le premier orifice de sortie 21 lorsque le récipient 2 est tête en bas, comme illustré sur la figure 4. Le clapet pourrait présenter toute autre forme appropriée, sans que l'on ne sorte du cadre de la présente invention.

Ce clapet peut permettre, lors du retrait de l'unité rechargeable 3, d'isoler le tube plongeur 17 de l'extérieur, de sorte que le produit contenu dans le tube plongeur 17 peut y demeurer.

La paroi de fond 14 comporte radialement à l'extérieur de la partie de raccordement 19 un deuxième orifice de sortie 25 dont le rôle sera expliqué plus loin.

L'unité rechargeable 3 comporte un corps 26 pourvu d'une paroi sensiblement cylindrique 27, d'axe X, se raccordant à une paroi de fond 28. La paroi 27 peut présenter une section transversale circulaire, elliptique ou autre.

Le corps 26 présente à sa partie inférieure un filetage 29 destiné à se visser dans la partie filetée 15 du support 9 lorsque l'unité rechargeable 3 est engagée dans le logement 24 de manière étanche.

La paroi de fond 28 présente extérieurement un renfoncement 39 dans lequel débouche un premier orifice 40 venant sensiblement en regard du premier orifice de sortie 21 du récipient 2 lorsque l'unité rechargeable 3 est en place dans le logement 24. La paroi de fond 28 comporte en outre un deuxième orifice 41, excentré par rapport à l'axe X, venant sensiblement en regard du deuxième orifice de sortie 25 du récipient 2.

Le corps 26 de l'unité rechargeable 3 comporte en partie inférieure au moins un relief 30a, tel que par exemple un bourrelet annulaire, apte à s'appliquer de façon étanche sur le support 9. Un relief 30b, tel que par exemple un bourrelet annulaire, est réalisé sur le corps 26 pour s'appliquer de façon sensiblement étanche sur la partie de raccordement 19. Les orifices 21 et 40 sont isolés de l'extérieur par les reliefs 30a et 30b.

Le corps 26 comporte, à son extrémité supérieure, un bourrelet annulaire 32 permettant la fixation d'un insert 33 définissant avec le corps 26 une chambre de stockage 35 de volume sensiblement constant.

L'insert 33 comporte une jupe de montage 36 coopérant avec le bourrelet 32 par encliquetage. En variante, l'insert 33 pourrait être fixé sur le corps 26 par vissage ou sertissage.

L'insert 33 comporte en outre une lèvre d'étanchéité 37 s'appliquant contre la surface intérieure du corps 26 et un col 38 de maintien d'une pompe 50, laquelle est dans l'exemple considéré conventionnelle et sans reprise d'air.

La pompe 50 peut comporter, comme illustré à la figure 3, un corps 51 fixé sur le col 38, par exemple par encliquetage.

Une tige de commande 52 est montée à coulissement dans le corps 51 contre l'action d'un ressort de rappel 53 travaillant en compression. Un embout 54 est fixé à l'extrémité inférieure de la tige de commande 52.

Un bouton-poussoir 55 servant à la fois d'organe d'actionnement et d'organe de distribution est emmanché à force sur l'extrémité supérieure de la tige de commande 52, comme on peut le voir sur la figure 2.

La tige de commande 52 comporte un évidement axial 58 s'étendant jusqu'à son extrémité supérieure et débouchant dans la chambre de pompage 57 par des orifices radiaux 55.

Un piston 56 est disposé à coulissement autour de la tige 52, ce piston 56 défmissant avec le corps 51 une chambre de pompage 57.

Le corps 51 forme un siège pour une bille 59 obturant au repos un orifice 60.

Lorsque la tige de commande 52 est au repos, les orifices 55 sont obturés par le piston 56.

Lorsque l'utilisateur appuie sur le bouton-poussoir 55, la tige de commande 52 s'enfonce dans le corps 51. Au début de la course d'enfoncement, le piston 56 n'est pas entraîné par la tige de commande 52. Cette dernière se déplace donc relativement au piston 56, ce qui libère les orifices 55. Lors de la poursuite de la course d'enfoncement de la tige de commande 52, cette dernière entraîne le piston 56 vers le bas. Le produit dans la chambre de pompage 57 est alors comprimé et s'écoule dans l'évidement 58 à travers les orifices 55, en vue d'être distribué.

Au cours de la descente de la tige de commande 52, la bille 59 reste en appui contre son siège dans le fond du corps 51 de manière à isoler la chambre de pompage 57 de la chambre de stockage 55.

Lorsque l'utilisateur relâche le bouton-poussoir 55, la tige de commande 52 coulisse tout d'abord dans le piston 56 jusqu'à venir en appui dessus par l'embout 54.

L'évidement axial 58 est alors isolé de la chambre de pompage 57 et la poursuite du mouvement de remontée de la tige de commande 52 sous l'action du ressort 53 provoque une dépression dans la chambre de pompage 57, laquelle s'accompagne du soulèvement de la bille 59 et de l'aspiration de produit dans la chambre de pompage 57.

Dans l'exemple considéré, la pompe 50 comporte un conduit 62 s'étendant jusqu'à la paroi de fond 28 du corps 26, ce conduit 62 présentant un orifice d'aspiration 63 débouchant à une distance très faible du premier orifice 40.

Le conduit 62 est maintenu à son extrémité inférieure par un embout 65 se raccordant à la paroi de fond 28.

Bien entendu, la pompe 50 peut présenter une structure différente sans que l'on sorte du cadre de la présente invention, la bille 59 pouvant notamment être remplacée par un clapet d'aspiration en élastomère par exemple.

La paroi de fond 28 de l'unité rechargeable 3 présente, sur sa face extérieure, une rainure 70 orientée radialement et définissant avec la partie de raccordement 19 du support 9 un passage 71, extérieur à la chambre de stockage 35. Ce passage 71 permet l'établissement d'une communication fluidique entre l'orifice d'aspiration 63 de la pompe 50 et la chambre de stockage 35, lorsque l'unité rechargeable 3 est en place sur le récipient 2.

La paroi de fond 28 se raccorde, à l'intérieur de la chambre de stockage 35, à des pattes 73 définissant un logement pour une bille 72 capable d'obturer le passage 71 lorsque l'unité rechargeable 3 est disposée sur le récipient 2, ce dernier étant tête en haut.

L'ensemble 1 peut s'utiliser de différentes façons.

Tout d'abord, l'ensemble 1 peut être utilisé dans une salle de bain par exemple pour prélever et distribuer du produit contenu dans le récipient 2.

A cet effet, l'utilisateur fixe l'unité rechargeable 3 dans le logement 24 du récipient 2.

Le filetage 29 de l'unité rechargeable 3 est engagé dans la partie filetée 15 du support 9 et les reliefs 30a, 30b, permettent d'isoler de manière étanche les orifices 21, 40, 25 et 41 de l'extérieur.

L'ensemble 1 est disposé tête en haut comme illustré sur la figure 2, de manière à ce que la bille 72 vienne obturer le passage 71. Ainsi, l'orifice d'aspiration 63 de la pompe 50 ne communique pas avec la chambre de stockage 35.

L'utilisateur actionne la pompe 50 en enfonçant le bouton-poussoir 55, ce qui provoque la distribution du produit présent dans la chambre de pompage 57.

Lorsque le bouton-poussoir 55 est relâché, une dépression se crée dans la chambre de pompage 57 de la pompe 50, provoquant ainsi l'aspiration par l'orifice d'aspiration 63 de produit provenant du récipient 2, qui s'écoule suivant un trajet passant par le tube plongeur 17, le logement 18 et les orifices 21 et 40, la bille 20 se soulevant pour permettre la circulation du produit.

Grâce à la bille 72, l'orifice d'aspiration 63 ne communique pas avec la chambre de stockage 35 mais uniquement avec la réserve de produit dans le récipient 2.

De plus, les reliefs 30a et 30b isolent l'orifice d'aspiration 63 de l'extérieur, ce qui évite un éventuel désamorçage de la pompe.

Lors de l'actionnement de la pompe, une reprise d'air est possible dans le récipient 2 par le passage 12.

L'unité rechargeable 3, préalablement rechargée, peut encore être utilisée séparément du récipient 2, et être transportée dans un sac à main par exemple.

Pour cela, l'utilisateur sépare l'unité rechargeable 3 du récipient 2 et fixe sur la paroi de fond 28 de l'unité rechargeable 3 un bouchon 80, comme illustré sur la figure 5.

On remarquera que lors de la séparation de l'unité rechargeable 3 du récipient 2, le produit ne s'échappe pas de cette chambre 35 par les orifices 40 et 41 avant la mise en place du bouchon 80, grâce à la tension superficielle du produit, l'unité rechargeable étant dépourvue d'orifice ouvert autre que ceux référencés 40 et 41.

Le bouchon 80 comporte une jupe de montage 81 permettant son vissage sur le filetage 19 du corps 26 et une partie saillante 82 s'engageant dans le renfoncement 39 du corps 26.

Le bouchon 80 comporte encore une rainure 83 définissant avec la paroi de fond 28 du corps 26 un passage 84 extérieur à la chambre de stockage 35, permettant de mettre en communication le deuxième orifice 41 avec le premier 40.

Un passage de reprise d'air 87 pour la chambre de stockage 35 est défini en outre entre le bouchon 80 et le corps 26.

Pour distribuer du produit contenu dans la chambre de stockage 35, l'utilisateur dispose l'unité rechargeable 3 tête en haut et actionne la pompe 50, ce qui permet l'aspiration par l'orifice d'aspiration 63 de produit contenu dans la chambre stockage 35, qui s'écoule suivant un trajet passant par l'orifice 41, le passage 83 et l'orifice 40.

Lorsque l'utilisateur relâche le bouton-poussoir 55, de l'air peut pénétrer dans la chambre de stockage 35 par le passage de reprise d'air 87.

Lorsque l'unité rechargeable 3 est séparée du récipient 2, il est possible de prévoir un capot, non représenté, permettant d'obturer les orifices de sortie 21 et 25 du récipient 2. Ce capot peut par exemple être agencé pour coiffer également le bouton-poussoir 55.

On notera qu'il est possible de fixer l'unité rechargeable 3 sur le récipient 2, même lorsque la chambre de stockage 35 n'est pas complètement vide.

Pour recharger la chambre de stockage 35, l'ensemble 1 est disposé tête en bas, comme illustré sur la figure 4.

Dans cette position, la bille 72 libère le passage 71 et l'orifice d'aspiration 63 de la pompe 50 peut communiquer avec la chambre de stockage 35, tandis que la bille 20 vient obturer l'orifice 21 de manière à isoler l'orifice d'aspiration 63 de la pompe 50 du tube plongeur 17.

L'utilisateur actionne la pompe 50, ce qui permet d'évacuer l'air dans la chambre de stockage 35 et d'y créer une dépression. Cette dernière provoque l'écoulement de produit du récipient 2 dans la chambre de stockage 35 à travers les orifices 25 et 41.

L'utilisateur peut être averti de la fin du remplissage de la chambre de stockage 35 lorsque l'actionnement de la pompe 50 provoque la distribution de produit.

On ne sort pas du cadre de la présente invention lorsque la chambre de stockage de l'unité rechargeable présente un volume variable.

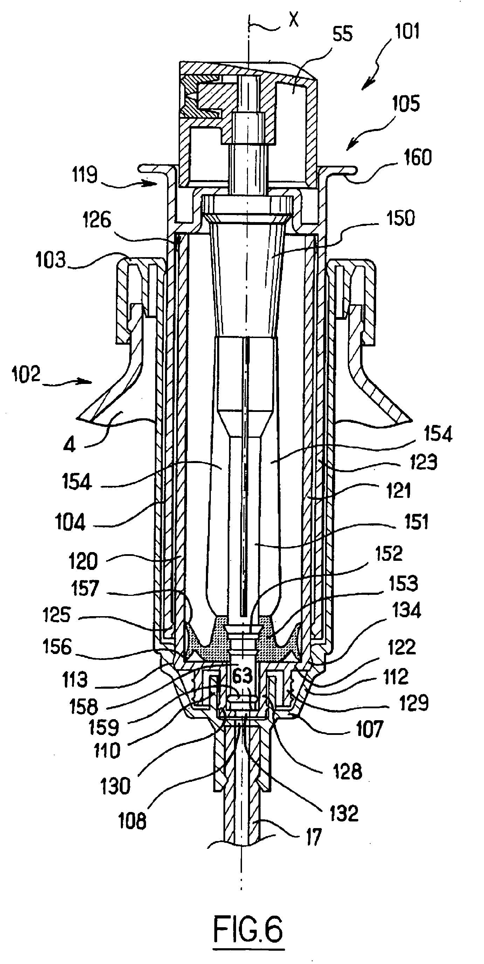

On va maintenant décrire en référence aux figures 6 à 8 un ensemble de conditionnement et de distribution 101 présentant une telle caractéristique.

L'ensemble 101 comporte un récipient 102 contenant une réserve de produit, notamment une crème. Le récipient 102 comprend un corps 4 et un support 103 fixé sur le corps 4 et définissant à l'instar du support 9 précédemment décrit un logement 104 pour recevoir une unité rechargeable 105.

Le support 103 se différencie du support 9 par le fait qu'il comporte une paroi de fond 107 traversée par un unique orifice de sortie 108, sur lequel débouche directement, sans l'intermédiaire d'un clapet, le tube plongeur 17.

La paroi de fond 107 se raccorde par sa face supérieure à une paroi sensiblement cylindrique 110, dans laquelle débouche l'orifice de sortie 108.

Le support 103 comporte en partie inférieure une portion intermédiaire 112 se raccordant à la paroi de fond 107, cette portion intermédiaire 112 comportant intérieurement une gorge 113.

L'unité rechargeable 105 comporte un corps 120 présentant une paroi sensiblement cylindrique 121 d'axe longitudinal X et une paroi de fond 122. La paroi 121 peut présenter une section transversale circulaire ou autre.

Un ensemble mobile 119 est déplaçable par rapport au corps 120, lequel comprend un fourreau extérieur 123. Ce dernier comporte sur sa surface intérieure, à son extrémité inférieure, des premiers reliefs 125 aptes à coopérer avec des seconds reliefs 126 réalisés à l'extrémité supérieure du corps 120 de manière à former une butée limitant la course vers le haut du fourreau 123 par rapport au corps 120.

Celui-ci comporte à son extrémité inférieure un embout 128 se raccordant à la paroi de fond 122 et, s'étendant autour de cet embout 128, une jupe de montage 129 filetée extérieurement, dont le rôle sera expliqué plus loin.

L'embout 128 comporte un bourrelet annulaire d'étanchéité 130 apte à s'appliquer de manière étanche contre la paroi 110 du récipient 102.

L'embout 128 est pourvu d'un orifice 132 venant se positionner au droit de l'orifice de sortie 108 du récipient 2, à une distance faible de celui-ci, voire sensiblement nulle, lorsque l'unité rechargeable 105 est en place sur le récipient 102, comme illustré sur la figure 6.

Le corps 120 de l'unité rechargeable 105 comporte, à l'extrémité inférieure de la paroi tubulaire 121, un bourrelet annulaire 134 apte à s'encliqueter dans la gorge 113 du support 103, afin de maintenir le corps 120 sur le support 103, comme cela sera expliqué plus loin.

L'ensemble mobile 119 comporte une pompe 150 sans reprise d'air, munie intérieurement d'une chambre de pompage.

La pompe 150 comporte un conduit 151 pourvu de reliefs de fixation 152 permettant la fixation d'un piston 153 autour du conduit 151. La face inférieure du piston 153 se situe en retrait de l'extrémité inférieure de ce conduit 151, ce dernier comportant une portion 158 se prolongeant vers le bas sous le piston 153, dont le rôle sera précisé plus loin.

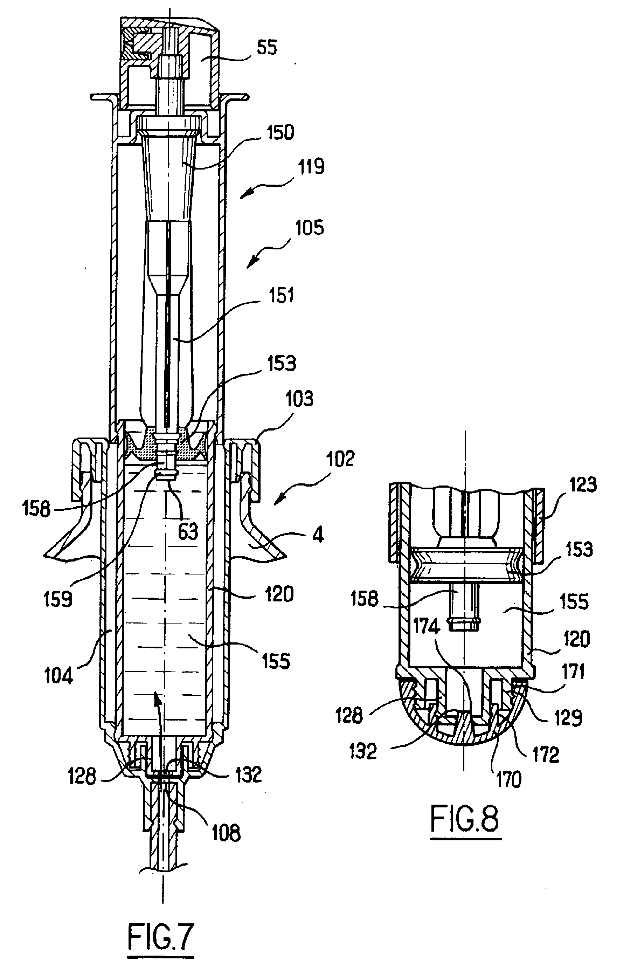

Le piston 153 comporte deux lèvres annulaires inférieure 156 et supérieure 157 venant en appui sur la surface intérieure du corps 120 et définit avec le corps 120, sous le piston 153, une chambre de stockage 155 de volume variable, comme on peut le voir sur les figures 7 et 8.

Le conduit 151 comporte en outre sur une majeure partie de sa hauteur des nervures longitudinales de rigidification 154 pouvant servir d'appui à la face supérieure du piston 153.

La portion de conduit 158 s'étendant sous le piston 153 comporte un bourrelet annulaire 159 apte à s'appliquer de manière étanche sur la surface intérieure de l'embout 132.

Le fourreau 123 est pourvu à son extrémité supérieure d'une collerette de préhension 160.

Afin de prélever et distribuer du produit contenu dans le récipient 102, l'utilisateur introduit l'unité rechargeable 105 dans le logement 104 du support 103, et enfonce l'ensemble mobile 119 jusqu'à sa position de fin de course, l'orifice d'aspiration 63 de la pompe 150 se trouvant alors au droit des orifices 108 et 132, à une distance sensiblement nulle de l'orifice 132. Le bourrelet annulaire 134 est alors engagé dans la gorge 113 du support 103. Dans cette position, les orifices 108 et 132 sont isolés de l'extérieur.

Ainsi, du produit peut s'écouler du tube plongeur 17 vers l'orifice d'aspiration 63 avec une perte de charge réduite.

Lorsque l'unité rechargeable 105 est disposée sur le récipient 102 avec la chambre de stockage 155 encore partiellement remplie de produit, l'ensemble mobile 119 n'est pas enfoncé jusqu'en position de fin de course dans le corps 120.

Pour raccorder le conduit 151 à l'orifice de sortie 108, l'utilisateur doit enfoncer l'ensemble mobile 119 jusqu'à sa position de fin de course, ce qui provoque l'expulsion du produit contenu dans la chambre de stockage 155 vers le récipient 102 à travers les orifices 132 et 108 et le tube plongeur 17. Durant cette étape, le bourrelet 130 est en appui étanche contre la paroi 110 du récipient 2, le bourrelet 134 n'étant pas encore engagé dans la gorge 113. En fin de course de l'ensemble mobile 119, la portion de conduit 158 s'engage dans l'embout 128, le volume de la chambre de stockage 155 étant alors minimal, étant par exemple sensiblement nul. La mise en place est terminée par l'engagement du bourrelet 134 dans la gorge 113. Cet engagement est ressenti par l'utilisateur, ce qui lui permet de savoir que la mise en place s'est réalisée correctement.

Une fois le conduit 151 raccordé à l'orifice de sortie 108, l'utilisateur peut prélever du produit à poste fixe dans le récipient 102, de préférence tête en haut, en actionnant la pompe 150.

Lorsque l'utilisateur appuie sur le bouton-poussoir 55, le produit aspiré par la pompe 150 suit un trajet passant par le tube plongeur 17 et les orifices 108 et 132.

Pour remplir la chambre de stockage 155 en vue d'une utilisation nomade de l'unité rechargeable, l'utilisateur fait coulisser l'ensemble mobile 119 par rapport au corps 120, vers le haut, en prenant appui sur le récipient 2 et en tirant avec ses doigts la collerette de préhension 160.

La coopération du bourrelet annulaire 134 avec la gorge 113 assure le maintien du corps 120 sur le support 103, contre une force de désengagement prédéfmie, supérieure à celle exercée sur ce corps 120 lorsque l'utilisateur fait coulisser vers le haut l'ensemble mobile 119. Ainsi, le corps 120 reste solidaire du support 103 lorsque l'ensemble mobile 119 est déplacé vers le haut.

Lors de cette opération, le volume de la chambre de stockage 155 augmente, ce qui crée dans celle-ci une dépression permettant l'aspiration de produit contenu dans le récipient 2, suivant un trajet passant par le tube plongeur 17 et les orifices 108 et 132.

Après le remplissage, l'utilisateur peut séparer l'unité rechargeable 105 du support 103 du récipient 102 en exerçant sur le corps 120 une force suffisante pour désengager le bourrelet annulaire 134 de la gorge 113.

Le produit contenu dans la chambre de stockage 155 ne fuit pas par l'orifice 132 compte tenu de la tension superficielle du produit.

Après séparation de l'unité rechargeable 105 et du récipient 102, l'utilisateur rapporte un bouchon 170 sur le corps 120. Ce bouchon 170 comporte un filetage 171 coopérant avec la jupe de montage 129 du corps 120, une lèvre d'étanchéité 172 et un picot central 174 destiné à s'engager dans l'orifice 132 du corps 120.

Le bouchon peut, en variante, présenter une autre forme.

Pour distribuer le produit contenu dans la chambre de stockage 155, l'utilisateur actionne la pompe 150.

Grâce à la présence de la portion de conduit 158 s'étendant sous le piston 153, la pompe 150 ne se désamorce pas même si une couche d'air s'est accumulée sous le piston 153, l'orifice d'aspiration 63 pouvant rester immergé dans le produit.

La pompe 150 étant sans reprise d'air, le corps 120 se déplace par rapport au piston 153 au fur et à mesure que le produit est distribué, comme illustré sur la figure 8.

Bien entendu, l'invention n'est pas limitée aux exemples de mise en oeuvre qui viennent d'être décrits.

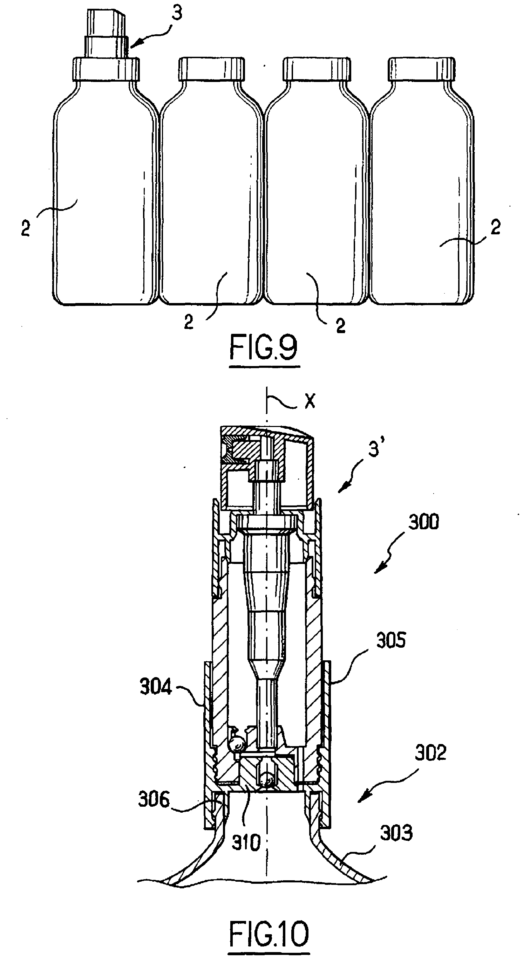

On peut utiliser une unité rechargeable, par exemple l'unité rechargeable 3 précédemment décrite, non pas avec un seul récipient 2 mais avec une pluralité de récipients 2 comme illustré sur la figure 9.

L'unité rechargeable 3 peut être disposée temporairement sur l'un de ces récipients 2 pour permettre le transfert d'une certaine quantité du produit du récipient 2 vers la chambre de stockage de l'unité rechargeable.

Chacun des récipients 2 peut ainsi contenir par exemple un produit correspondant à une senteur particulière, et l'utilisateur peut sélectionner l'un des récipients en fonction de la senteur qu'il souhaite utiliser dans la journée.

L'utilisateur peut également réaliser un mélange de produits prélevés successivement dans plusieurs récipients 2.

Les récipients 2 peuvent être vendus séparément de l'unité rechargeable 3.

On a représenté sur la figure 10 un ensemble de conditionnement et de distribution 300 conforme à un autre exemple de mise en oeuvre de l'invention.

Cet ensemble 300 comporte une unité rechargeable 3' sensiblement analogue à celle précédemment décrite.

L'unité rechargeable 3' peut présenter une hauteur moindre et une section transversale plus grande que l'unité rechargeable 3.

L'unité rechargeable 3' peut être rechargée à l'aide d'un récipient 302 comportant un corps de récipient 303 avec un col 306 sur lequel est fixé, par exemple par encliquetage, un support 304.

Le support 304 comporte par exemple une jupe tubulaire 305 d'axe X s'étendant au-dessus du col 306 du corps de récipient 303 pour recevoir l'unité rechargeable 3'.

Dans l'exemple considéré, le support 304 comporte une paroi de fond 310 sensiblement analogue à la paroi de fond 14 précédemment décrite, avec la différence que la paroi 310 est dépourvue d'embout pour la fixation du tube plongeur.

Le récipient 302 peut être sans reprise d'air et comporter un piston ou une poche non représentés, au contact du produit et permettant d'accompagner la réduction du volume de la réserve de produit contenue dans le récipient 2 après chaque prélèvement de produit par l'unité rechargeable.

Dans toute la description, y compris les revendications, l'expression « comportant un » doit être comprise comme étant synonyme de « comportant au moins un », sauf si le contraire est spécifié.

## Revendications

1. Ensemble (1 ; 101 ; 201) de conditionnement et de distribution d'un produit, comportant :
- un récipient (2 ; 102 ; 302) pour contenir une réserve de produit,
- une unité rechargeable (3 ; 105 ; 3') agencée pour être disposée de façon amovible sur le récipient, l'unité rechargeable comportant :
- une chambre de stockage (35 ; 155) pour contenir du produit et capable d'être mise en communication fluidique avec le récipient pour son remplissage lorsque l'unité rechargeable est disposée sur le récipient, **caractérisé par**
- une pompe (50 ; 150) capable de prélever du produit dans le récipient lorsque l'unité rechargeable est disposée dessus, la pompe comportant un orifice d'aspiration (63) permettant une communication fluidique entre l'orifice d'aspiration (63) et le récipient, et capable de prélever du produit dans la chambre de stockage (35 ; 155) lorsque l'unité rechargeable est séparée du récipient, l'orifice d'aspiration (63) permettant une communication fluidique entre l'orifice d'aspiration (63) et la chambre de stockage, la pompe comportant en outre une chambre de pompage (57) distincte de la chambre de stockage (35 ; 155).

2. Ensemble selon la revendication précédente, **caractérisé par le fait que** l'unité rechargeable (3 ; 3' ; 105) est agencée pour permettre de générer une dépression dans la chambre de stockage, au moins lorsque la chambre de stockage (35 ; 155) est en communication fluidique avec le récipient, de manière à permettre le remplissage de la chambre de stockage.

3. Ensemble selon l'une des deux revendications précédentes, **caractérisé par le fait que** le récipient comporte un orifice de sortie (21 ; 132) apte à être mis en communication fluidique avec la chambre de stockage (35 ; 155) de l'unité rechargeable pour son remplissage, et **par le fait que** le récipient est dépourvu de pompe capable d'alimenter en produit ledit orifice de sortie (21 ; 132).

4. Ensemble selon l'une quelconque des revendications précédentes, **caractérisé par le fait que** la chambre de pompage (57) est isolée de la chambre de stockage (35 ; 155) au moins lors de la distribution de produit par la pompe.

5. Ensemble selon l'une quelconque des revendications précédentes, **caractérisé par le fait que** le volume de la chambre de pompage (57) est inférieur au volume maximal de la chambre de stockage (35 ; 155).

6. Ensemble selon l'une quelconque des revendications précédentes, la pompe comportant un orifice d'aspiration (63), **caractérisé par le fait que**, lorsque l'unité rechargeable est disposée sur le récipient, ledit orifice d'aspiration (63) de la pompe (50 ; 150) est apte à être disposé à proximité d'un orifice de sortie (21 ; 132) du récipient, à l'aplomb de cet orifice de sortie.

7. Ensemble selon l'une quelconque des revendications précédentes, **caractérisé par le fait que** la pompe (50 ; 150) est une pompe sans reprise d'air.

8. Ensemble selon l'une quelconque des revendications précédentes, **caractérisé par le fait que** le récipient comporte un support (9 ; 103 ; 304) agencé pour recevoir l'unité rechargeable, ledit support (9 ; 103) étant immobile par rapport au reste du récipient.

9. Ensemble selon l'une quelconque des revendications précédentes, **caractérisé par le fait que** l'unité rechargeable et le récipient comportent des parties de fixation (15, 29 ; 113, 134) respectives aptes à coopérer de manière amovible, notamment par encliquetage ou vissage.

10. Ensemble selon l'une quelconque des revendications précédentes, **caractérisé par le fait que** l'un au moins de l'unité rechargeable et du récipient comporte des reliefs (30a ; 30b ; 111) aptes à coopérer avec l'autre de l'unité rechargeable et du récipient pour isoler de manière étanche l'orifice d'aspiration (63) de la pompe de l'extérieur.

11. Ensemble selon l'une quelconque des revendications précédentes, **caractérisé par le fait que** le récipient comporte un passage de reprise d'air (12).

12. Ensemble selon l'une quelconque des revendications 1 à 10, **caractérisé par le fait que** le récipient est sans reprise d'air.

13. Ensemble selon l'une quelconque des revendications précédentes, **caractérisé par le fait que** la chambre de stockage (35) présente un volume sensiblement constant.

14. Ensemble selon la revendication précédente, **caractérisé par le fait que** l'unité rechargeable (3) comporte un premier orifice (40) apte à être mis en communication fluidique avec la pompe (50) et le récipient (2) pour permettre à la pompe de prélever du produit dans le récipient lorsque l'unité rechargeable (3) est disposée sur le récipient, et un deuxième orifice (41) distinct du premier, apte à être mis en communication fluidique avec le récipient (2) pour le remplissage de la chambre de stockage.

15. Ensemble selon la revendication précédente, **caractérisé par le fait que** le deuxième orifice (41) est réalisé sur une paroi de fond (28) de l'unité rechargeable.

16. Ensemble selon l'une quelconque des revendications 13 à 15, la pompe comportant un orifice d'aspiration (63), **caractérisé par le fait que** l'unité rechargeable (3) comporte un clapet (72) capable d'isoler l'orifice d'aspiration (63) de la chambre de stockage (35) lorsque l'unité rechargeable (3) est disposée sur le récipient (2), ce dernier étant tête en haut, et de permettre une communication fluidique entre ledit orifice d'aspiration (63) et la chambre de stockage (35) lorsque l'unité rechargeable (3) est disposée sur le récipient (2), ce dernier étant tête en bas.

17. Ensemble selon la revendication précédente, **caractérisé par le fait que** l'unité rechargeable (3) comporte une paroi, notamment une paroi de fond (28), agencée pour former, avec une paroi du récipient (19), lorsque l'unité rechargeable est disposée sur le récipient, un passage (71) à l'extérieur de la chambre de stockage (35), pouvant mettre en communication fluidique l'orifice d'aspiration (63) de la pompe avec la chambre de stockage (35), ce passage pouvant être obturé par ledit clapet (72).

18. Ensemble selon la revendication 14 et éventuellement l'une quelconque des revendications 15 à 17, **caractérisé par le fait que** le récipient (2) comporte un premier orifice de sortie (21) apte à être mis en communication fluidique avec le premier orifice (40) de l'unité rechargeable (3) lorsque l'unité rechargeable est disposée sur le récipient, et un deuxième orifice de sortie (25) apte à être mis en communication fluidique avec le deuxième orifice (41) de l'unité rechargeable (3) lorsque l'unité rechargeable est disposée sur le récipient.

19. Ensemble selon la revendication précédente, **caractérisé par le fait que** le récipient comporte un clapet (20) capable d'obturer ledit premier orifice de sortie (21) du récipient (2) lorsque le récipient est tête en bas, et de le libérer lorsque le récipient est tête en haut.

20. Ensemble selon la revendication 14 et éventuellement l'une quelconque des revendications 15 à 19, **caractérisé par le fait que** l'unité rechargeable (3) comporte un corps (26) sur lequel sont réalisés les premier (40) et deuxième (41) orifices et un bouchon (80) agencé pour être fixé de manière amovible sur ledit corps (26) lorsque l'unité rechargeable (3) est séparée du récipient, afin d'obturer lesdits premier (40) et deuxième (41) orifices.

21. Ensemble selon la revendication précédente, **caractérisé par le fait que** le bouchon (80) forme avec ledit corps (26), lorsqu'ils sont assemblés, un passage (84) à l'extérieur de la chambre de stockage entre les premier (40) et deuxième (41) orifices, permettant de mettre en communication fluidique la pompe (50) avec la chambre de stockage (35).

22. Ensemble selon l'une des deux revendications précédentes, **caractérisé par le fait que** le bouchon (80) définit avec ledit corps (26), lorsqu'ils sont assemblés, un passage de reprise d'air.

23. Ensemble selon l'une quelconque des revendications 1 à 12, **caractérisé par le fait que** l'unité rechargeable (105) comporte un corps (120) et un piston (153) mobile par rapport au corps (120) et définissant avec celui-ci une chambre de stockage de volume variable (155).

24. Ensemble selon la revendication précédente, **caractérisé par le fait que**, lorsque l'unité rechargeable (105) est disposée sur le récipient (102) de manière à ce que la pompe puisse prélever du produit dans le récipient, la pompe communiquant notamment avec un tube plongeur (17) du récipient, le volume de la chambre de stockage (155) est minimal.

25. Ensemble selon l'une des deux revendications précédentes, **caractérisé par le fait que** la chambre de stockage (155) est formée sous le piston (153), lorsque l'unité rechargeable est observée tête en haut.

26. Ensemble selon la revendication précédente, **caractérisé par le fait que** l'unité rechargeable (105) comporte un ensemble mobile (119) par rapport au corps (120), l'ensemble mobile (119) comprenant le piston (153).

27. Ensemble selon la revendication précédente, **caractérisé par le fait que** l'ensemble mobile (119) comporte un fourreau extérieur (123) coulissant sur le corps (120).

28. Ensemble selon l'une des deux revendications précédentes, **caractérisé par le fait que** l'ensemble mobile (119) comporte une pompe (150).

29. Ensemble selon la revendication précédente, **caractérisé par le fait que** la pompe (150) comporte un conduit (151), le piston (153) étant fixé autour dudit conduit.

30. Ensemble selon la revendication précédente, **caractérisé par le fait que** le piston (153) présente une face inférieure en retrait de l'extrémité inférieure du conduit (151), ce dernier comportant une portion (158) se prolongeant vers le bas sous le piston (153).

31. Ensemble selon l'une quelconque des revendications 25 à 30, **caractérisé par le fait que** le corps (120) de l'unité rechargeable (105) comporte un orifice (132) permettant de mettre en communication fluidique la chambre de stockage (155) avec le récipient (102).

32. Ensemble selon la revendication précédente, **caractérisé par le fait que** l'unité rechargeable (105) comporte un bouchon (170) apte à être fixé de manière amovible, notamment par vissage ou encliquetage, sur le corps (120) pour obturer ledit orifice (132) lorsque l'unité rechargeable (105) est séparée du récipient (102).

33. Ensemble selon l'une quelconque des revendications précédentes, **caractérisé par le fait que** le récipient contient un produit cosmétique, y compris de soin, notamment un parfum.

34. Ensemble selon la revendication précédente, **caractérisé par le fait que** le produit est une crème.

35. Procédé de distribution d'un produit, comportant les étapes suivantes :
- fournir un ensemble (1 ; 101) de conditionnement et de distribution, comportant :
- un récipient (2 ; 102 ; 3') pour contenir une réserve de produit,
- une unité rechargeable (3 ; 105) agencée pour être disposée de façon amovible sur le récipient et comportant une chambre de stockage (35 ; 155) et une pompe (50 ; 150), **caractérisé par** les étapes suivantes:
- prélever du produit à l'aide de l'unité rechargeable soit dans le récipient lorsque l'unité rechargeable est disposée sur le récipient, soit dans la chambre de stockage de l'unité rechargeable lorsque l'unité rechargeable est séparée du récipient,
- distribuer une dose de produit prélevée à l'aide de l'unité rechargeable en actionnant la pompe sur une course complète, la dose délivrée correspondant à une fraction seulement du volume maximal de la chambre de stockage, notamment à moins du dixième.

36. Procédé selon la revendication précédente, la pompe comportant un orifice d'aspiration (63) et le récipient un orifice de sortie (21 ; 132), **caractérisé par le fait que** le procédé comporte les étapes suivantes :
- mettre l'orifice d'aspiration (63) en communication avec l'orifice de sortie (21 ; 132),
- prélever du produit dans le récipient.

37. Procédé selon l'une des deux revendications précédentes, **caractérisé par le fait qu'**il comporte les étapes suivantes :
- disposer l'unité rechargeable (3) sur le récipient (2), l'ensemble étant tête en bas,
- actionner la pompe (50) à plusieurs reprises sur une course complète, pour créer une dépression dans la chambre de stockage et permettre le remplissage en produit de celle-ci, sans que le produit ne soit distribué entre deux courses complètes.

38. Procédé selon la revendication 36, l'unité rechargeable comportant un corps et un piston coulissant dans le corps, **caractérisé par le fait que** le procédé comporte les étapes suivantes :
- disposer l'unité rechargeable (105) sur le récipient (102),
- déplacer le piston relativement au corps afin d'augmenter le volume de la chambre de stockage, le piston étant lié fixement à la pompe lors de ce déplacement.

## Claims

1. An assembly (1; 101; 201) for packaging and dispensing liquid, the assembly comprising:
• a receptacle (2; 102; 302) for containing a supply of liquid; and
• a refillable unit (3; 105; 3') arranged to be disposed in separable manner on the receptacle, the refillable unit comprising:
• a storage chamber (35; 155) for containing the liquid and capable of being put into fluid communication with the receptacle in order to be filled therefrom when the refillable unit is placed on the receptacle, the assembly being **characterized in that**
• a pump (50; 150) capable of taking liquid from the receptacle when the refillable unit is placed thereon, the pump comprising a suction orifice (63) allowing for fluid communication between the suction orifice (63) and the receptacle, and capable of taking liquid from the storage chamber (35; 155) when the refillable unit is separate from the receptacle, the suction orifice (63) allowing for fluid communication between the suction orifice (63) and the storage chamber, the pump further having a pump chamber (57) that is distinct from the storage chamber (35; 155).

2. An assembly according to the preceding claim, **characterized by** the fact that the refillable unit (3; 3'; 105) is arranged to enable suction to be generated in the storage chamber, at least when the storage chamber (35; 155) is in fluid communication with the receptacle, so as to enable the storage chamber to be filled.

3. An assembly according to either one of the two preceding claims, **characterized by** the fact that the receptacle has an outlet orifice (21; 132) suitable for being put into fluid communication with the storage chamber (35; 155) of the refillable unit for filling purposes, and by the fact that the receptacle does not have a pump capable of feeding said outlet orifice (21; 132) with liquid.

4. An assembly according to any preceding claim, **characterized by** the fact that the pump chamber (57) is isolated from the storage chamber (35; 155), at least while the pump is dispensing liquid.

5. An assembly according to any preceding claim, **characterized by** the fact that the volume of the pump chamber (57) is smaller than the maximum volume of the storage chamber (35; 155).

6. An assembly according to any preceding claim, the pump including a suction orifice (63), the assembly being **characterized by** the fact that, when the refillable unit is placed on the receptacle, said suction orifice (63) of the pump (50; 150) is suitable for being placed close to an outlet orifice (21; 132) of the receptacle, vertically in register with said outlet orifice.

7. An assembly according to any preceding claim, **characterized by** the fact that the pump (50; 150) is an airless pump.

8. An assembly according to any preceding claim, **characterized by** the fact that the receptacle includes a support (9; 103; 304) arranged to receive the refillable unit, said support (9; 103) being stationary relative to the remainder of the receptacle.

9. An assembly according to any preceding claim, **characterized by** the fact that the refillable unit and the receptacle have respective fastener portions (15, 29; 113, 134) suitable for co-operating in releasable manner, in particular by snap-fastening or by screw-fastening.

10. An assembly according to any preceding claim, **characterized by** the fact that at least one of the refillable unit and of the receptacle includes portions in relief (30a; 30b; 111) suitable for co-operating with the other one of the refillable unit and the receptacle in order to isolate the suction orifice (63) of the pump in leaktight manner from the outside.

11. An assembly according to any preceding claim, **characterized by** the fact that the receptacle includes an air intake passage (12).

12. An assembly according to any one of claims 1 to 10, **characterized by** the fact that the receptacle does not have an air intake.

13. An assembly according to any preceding claim, **characterized by** the fact that the storage chamber (35) presents a volume that is substantially constant.

14. An assembly according to the preceding claim, **characterized by** the fact that the refillable unit (3) has a first orifice (40) suitable for being put into fluid communication with the pump (50) and the receptacle (2) in order to enable the pump to take liquid from the receptacle when the refillable unit (3) is placed on the receptacle, and a second orifice (41) distinct from the first orifice, suitable for being put into fluid communication with the receptacle (2) in order to fill the storage chamber.

15. An assembly according to the preceding claim, **characterized by** the fact that the second orifice (41) is made in a bottom wall (29) of the refillable unit.

16. An assembly according to any one of claims 13 to 15, in which the pump includes a suction orifice (63), the assembly being **characterized by** the fact that the refillable unit (3) includes a check valve (72) suitable for isolating the suction orifice (63) of the storage chamber (35) when the refillable unit (3) is placed on the receptacle (2), the receptacle being in the head-up position, and of allowing fluid communication between said suction orifice (63) and the storage chamber (35) when the refillable unit (3) is placed on the receptacle (2), with the receptacle in the head-down position.

17. An assembly according to the preceding claim, **characterized by** the fact that the refillable unit (3) has a wall, in particular a bottom wall (28), arranged to co-operate with a wall of the receptacle (19) when the refillable unit is placed on the receptacle, to form a passage (71) outside the storage chamber (35) capable of putting the suction orifice (63) of the pump into fluid communication with the storage chamber (35), said passage being closable by said check valve (72).

18. An assembly according to claim 14, and optionally according to any one of claims 15 to 17, **characterized by** the fact that the receptacle (2) has a first outlet orifice (21) suitable for being put into fluid communication with the first orifice (40) of the refillable unit (3) when the refillable unit is placed on the receptacle, and a second outlet orifice (25) suitable for being put into fluid communication with the second orifice (41) of the refillable unit (3) when the refillable unit is placed on the receptacle.

19. An assembly according to the preceding claim, **characterized by** the fact that the receptacle includes a check valve (20) capable of closing said first outlet orifice (21) of the receptacle (2) when the receptacle is in a head-down position, and of releasing said orifice when the receptacle is in a head-up position.

20. An assembly according to claim 14, and optionally according to any one of claims 15 to 19, **characterized by** the fact that the refillable unit (3) has a body (26) having the first and second orifices (40 and 41) formed therein, and a cap (80) arranged to be releasably secured on said body (26) when the refillable unit (3) is separate from the receptacle, in order to close said first and second orifices (40 and 41).

21. An assembly according to the preceding claim, **characterized by** the fact that the cap (80) co-operates with the said body (26), when they are assembled together, to form a passage (84) outside the storage chamber between the first and second orifices (40 and 41), enabling the pump (50) to be put into fluid communication with the storage chamber (35).

22. An assembly according to either one of the two preceding claims, **characterized by** the fact that the cap (80) co-operates with said body (26), when they are assembled together, to define an air intake passage.

23. An assembly according to any one of claims 1 to 12, **characterized by** the fact that the refillable unit (105) includes a body (120) and a piston (153) that is movable relative to the body (120) and that co-operates therewith to define a storage chamber (155) of variable volume.

24. An assembly according to the preceding claim, **characterized by** the fact that, when the refillable unit (105) is placed on the receptacle (102) so that the pump can take liquid from the receptacle, with the pump communicating in particular with a dip tube (17) of the receptacle, the volume of the storage chamber (155) is at its minimum.

25. An assembly according to either one of the two preceding claims, **characterized by** the fact that the storage chamber (155) is formed under the piston (153) when the refillable unit is observed in the head-up position.

26. An assembly according to the preceding claim, **characterized by** the fact that the refillable unit (105) includes a moving assembly (119) that is movable relative to the body (120), the moving assembly (119) including the piston (153).

27. An assembly according to the preceding claim, **characterized by** the fact that the moving assembly (119) includes an outer sheath (123) slidable on the body (120).

28. An assembly according to either one of the two preceding claims, **characterized by** the fact that the moving assembly (119) includes a pump (150).

29. An assembly according to the preceding claim, **characterized by** the fact that the pump (150) includes a duct (151), with the piston (153) being secured around said duct.

30. An assembly according to the preceding claim, **characterized by** the fact that the piston (153) presents a bottom face that is set back from the bottom end of the duct (151), which duct has a portion (158) extending it downwards beneath the piston (153).

31. An assembly according to any one of claims 25 to 30, **characterized by** the fact that the body (120) of the refillable unit (105) has an orifice (132) enabling the storage chamber (155) to be put into fluid communication with the receptacle (102).

32. An assembly according to the preceding claim, **characterized by** the fact that the refillable unit (105) includes a cap (170) suitable for being fastened in releasable manner, in particular by screw-fastening or by snap-fastening, to the body (120) so as to close said orifice (132) when the refillable unit (105) is separate from the receptacle (102).

33. An assembly according to any preceding claim, **characterized by** the fact that the receptacle contains a cosmetic, including a care product, and in particular a perfume.

34. An assembly according to the preceding claim, **characterized by** the fact that the liquid is a cream.

35. A method of dispensing liquid, the method comprising the following steps:
• providing a packaging and dispenser assembly (1; 101), comprising:
• a receptacle (2; 102; 3') for containing a supply of liquid; and
• a refillable unit (3; 105) arranged to be placed in separable manner on the receptacle and comprising a storage chamber (35; 155) and a pump (50; 150), the method being **characterized by** the following steps:
• using the refillable unit to take liquid either from the receptacle when the refillable unit is placed on the receptacle, or from the storage chamber of the refillable unit when the refillable unit is separate from the receptacle; and
• dispensing a quantity of liquid taken by means of the refillable unit by actuating the pump over a full stroke, the quantity of liquid dispensed corresponding to a fraction only of the maximum volume of the storage chamber, and in particular to less than one-tenth thereof.

36. A method according to the preceding claim, in which the pump includes a suction orifice (63) and the receptacle includes an outlet orifice (21; 132), the method being **characterized by** the fact that it includes the following steps:
• putting the suction orifice (63) into communication with the outlet orifice (21; 132); and
• taking liquid from the receptacle.

37. A method according to either one of the two preceding claims, **characterized by** the fact that it includes the following steps:
• placing the refillable unit (3) on the receptacle (2), the assembly being in the head-down position; and
• actuating the pump (50) several times over a full stroke in order to establish suction in the storage chamber and allow it to be filled with liquid without any liquid being dispensed between two full strokes.

38. A method according to claim 36, in which the refillable unit includes a body and a piston slidable in the body, the method being **characterized by** the fact that it includes the following steps:
• placing the refillable unit (105) on the receptacle (102); and
• moving the piston relative to the body in order to increase the volume of the storage chamber, the piston being stationary relative to the pump during this movement.

## Patentansprüche

1. Aufbau (1; 101; 201) zur Aufbereitung und zur Verteilung von einem Produkt, der aufweist:
- einen Behälter (2; 102; 302), um einen Vorrat des Produktes zu enthalten,
- eine nachfüllbare Einheit (3; 105; 3'), die angeordnet ist, um in einer bewegbaren Weise auf dem Behälter platziert zu sein, wobei die nachfüllbare Einheit aufweist:
- eine Vorratskammer (35; 155), um das Produkt zu enthalten und dazu in der Lage zu sein, um in eine Fluidverbindung mit dem Behälter für seine Auffüllung gebracht zu werden, wenn die nachfüllbare Einheit auf dem Behälter angeordnet ist, **gekennzeichnet durch**
- eine Pumpe (50; 150), die dazu in der Lage ist, das Produkt in dem Behälter zu entnehmen, wenn die nachfüllbare Einheit darauf angeordnet ist, wobei die Pumpe aufweist, eine Ansaugöffnung (63), die eine Fluidverbindung zwischen der Ansaugöffnung (63) und dem Behälter ermöglicht, und dazu in der Lage ist, Produkt in der Vorratskammer (35; 155) zu entnehmen, wenn die nachfüllbare Einheit von dem Behälter getrennt ist, wobei die Ansaugöffnung (63) eine Fluidverbindung zwischen der Ansaugöffnung (63) und der Vorratskammer ermöglicht, wobei die Pumpe ferner eine Pumpkammer (57) aufweist, die sich von der Vorratskammer (35; 155) unterscheidet.

2. Aufbau nach dem voranstehenden Anspruch, **dadurch gekennzeichnet, dass** die nachfüllbare Einheit (3; 3'; 105) angeordnet ist, um es zu ermöglichen, in der Vorratskammer einen Unterdruck zu erzeugen, zumindest wenn die Vorratskammer (35; 155) in Fluidverbindung mit dem Behälter ist, in der Weise, um das Auffüllen der Vorratskammer zu ermöglichen.

3. Aufbau nach einem der zwei voranstehenden Ansprüche, **dadurch gekennzeichnet, dass** der Behälter eine Ausgangsöffnung (21; 132) aufweist, die geeignet ist, um in Fluidverbindung mit der Vorratskammer (35; 155) der nachfüllbaren Einheit für deren Befüllung gebracht zu werden, und **dadurch**, dass der Behälter ohne Pumpe dazu in der Lage ist, die Ausgangsöffnung (21; 132) mit dem Produkt zu versorgen.

4. Aufbau nach irgendeinem der voranstehenden Ansprüche, **dadurch gekennzeichnet, dass** die Pumpkammer (57) von der Vorratskammer (35; 155) zumindest während der Verteilung des Produktes durch die Pumpe getrennt ist.

5. Aufbau nach irgendeinem der voranstehenden Ansprüche, **dadurch gekennzeichnet, dass** das Volumen der Pumpkammer (57) geringer als das maximale Volumen der Vorratskammer (35; 155) ist.

6. Aufbau nach irgendeinem der voranstehenden Ansprüche, wobei die Pumpe aufweist, eine Auslassöffnung (63), **dadurch gekennzeichnet, dass** wenn die nachfüllbare Einheit auf dem Behälter angeordnet ist, die Auslassöffnung (63) der Pumpe (50; 150) dazu geeignet ist, in der Nähe einer Ausgangsöffnung (21; 132) des Behälters in der senkrechten Stellung von dieser Ausgangsöffnung angeordnet zu sein.

7. Aufbau nach irgendeinem der voranstehenden Ansprüche, **dadurch gekennzeichnet, dass** die Pumpe (50; 150) eine Pumpe ohne Luftnachführung ist.

8. Aufbau nach irgendeinem der voranstehenden Ansprüche, **dadurch gekennzeichnet, dass** der Behälter einen Träger (9; 103; 304) aufweist, der angeordnet ist, um die nachfüllbare Einheit aufzunehmen, wobei der Träger (9; 103) im Hinblick auf den Rest des Behälters unbeweglich bzw. fest ist.

9. Aufbau nach irgendeinem der voranstehenden Ansprüche, **dadurch gekennzeichnet, dass** die nachfüllbare Einheit und der Behälter Abschnitte zur Festlegung (15, 29; 113, 134) aufweisen, die jeweils dazu in der Lage sind, in einer beweglichen Weise miteinander in Wirkverbindung zu treten, insbesondere durch Verrastung oder Verschraubung.

10. Aufbau nach irgendeinem der voranstehenden Ansprüche, **dadurch gekennzeichnet, dass** zumindest einer von der nachfüllbaren Einheit und dem Behälter Strukturen (30a; 30b; 111) aufweist, die dazu in der Lage sind, mit dem Gegenstück der nachfüllbaren Einheit und des Behälters zu kooperieren, um in einer dichtenden Weise die Auslassöffnung (63) der Pumpe von dem Äußeren zu trennen.

11. Aufbau nach irgendeinem der voranstehenden Ansprüche, **dadurch gekennzeichnet, dass** der Behälter einen Durchgang zur Luftnachführung (12) aufweist.

12. Aufbau nach irgendeinem der Ansprüche 1 bis 10, **dadurch gekennzeichnet, dass** der Behälter ohne Luftnachführung ist.

13. Aufbau nach irgendeinem der voranstehenden Ansprüche, **dadurch gekennzeichnet, dass** die Vorratskammer (35) ein genau konstantes Volumen vorzuweisen hat.

14. Anordnung nach dem voranstehenden Anspruch, **dadurch gekennzeichnet, dass** die nachfüllbare Einheit (3) aufweist, eine erste Öffnung (40), die dazu geeignet ist, um in Fluidverbindung mit der Pumpe (50) und dem Behälter (2) gebracht zu werden, um der Pumpe die Entnahme von Produkt in dem Behälter zu ermöglichen, wenn die nachfüllbare Einheit (3) auf dem Behälter angeordnet ist, und eine zweite Öffnung (41), die sich von der ersten unterscheidet, die dafür geeignet ist, um in Fluidverbindung mit dem Behälter (2) für die Befüllung der Vorratskammer gebracht zu werden.

15. Aufbau nach dem voranstehenden Anspruch, **dadurch gekennzeichnet, dass** die zweite Öffnung (41) auf einer Bodenwand (28) der nachfüllbaren Einheit ausgebildet ist.

16. Aufbau nach irgendeinem der Ansprüche 13 bis 15, wobei die Pumpe aufweist, eine Auslassöffnung (63), **dadurch gekennzeichnet, dass** die nachfüllbare Einheit (3) aufweist, einen Verschluss bzw. eine Klappe (72), die dazu in der Lage ist, die Auslassöffnung (63) von der Vorratskammer (35) zu trennen, wenn die nachfüllbare Einheit (3) auf dem Behälter (2) angeordnet ist, wobei letzterer mit dem Kopf nach oben ist, und eine Fluidverbindung zwischen der Auslassöffnung (63) und der Vorratskammer (35) zu ermöglichen, wenn die nachfüllbare Einheit (3) auf dem Behälter (2) angeordnet ist, wobei letzterer mit dem Kopf nach unten ist.

17. Aufbau nach irgendeinem der voranstehenden Ansprüche, **dadurch gekennzeichnet, dass** die nachfüllbare Einheit (3) aufweist, eine Wand, insbesondere eine Bodenwand (28), die angeordnet ist, um mit einer Wand des Behälters (19), wenn die nachfüllbare Einheit auf dem Behälter angeordnet ist, einen Durchgang (71) nach dem Äußeren der Vorratskammer (35) auszubilden, wobei eine Fluidverbindung der Auslassöffnung (63) von der Pumpe mit der Vorratskammer (35) hergestellt werden kann, wobei dieser Durchgang von der Klappe (72) bzw. dem Verschluss verschlossen werden kann.

18. Aufbau nach Anspruch 14 und gegebenenfalls nach irgendeinem der Ansprüche 15 bis 17, **dadurch gekennzeichnet, dass** der Behälter (2) aufweist, eine erste Ausgangsöffnung (21), die dazu geeignet ist, in eine Fluidverbindung mit der ersten Öffnung (40) der nachfüllbaren Einheit (3) gebracht zu werden, wenn die nachfüllbare Einheit auf dem Behälter angeordnet ist, und eine zweite Ausgangsöffnung (25), die dazu geeignet ist, um in Fluidverbindung mit der zweiten Öffnung (41) der nachfüllbaren Einheit (3) gebracht zu werden, wenn die nachfüllbare Einheit auf dem Behälter angeordnet ist.

19. Aufbau nach dem voranstehenden Anspruch, **dadurch gekennzeichnet, dass** der Behälter aufweist, einen Verschluss (20), der dazu in der Lage ist, die erste Ausgangsöffnung (21) des Behälters (2) zu verschließen, wenn der Behälter mit dem Kopf nach unten ist, und diesen freizugeben, wenn der Behälter mit dem Kopf nach oben ist.

20. Aufbau nach Anspruch 14 und gegebenenfalls nach einem der Ansprüche 15 bis 19, **dadurch gekennzeichnet, dass** die nachfüllbare Einheit (3) aufweist, einen Körper (26), auf welchem die erste (40) und die zweite Öffnung (41) und ein Deckel (80), der angeordnet ist, um in einer bewegbaren Weise auf dem Körper (26) angeordnet zu sein, wenn die nachfüllbare Einheit (3) von dem Behälter getrennt ist, verwirklicht sind, um die erste (40) und die zweite Öffnung (40) zu verschließen.

21. Aufbau nach dem voranstehenden Anspruch, **dadurch gekennzeichnet, dass** der Deckel (80) mit dem Körper (26), wenn diese verbunden sind, einen Durchgang (84) nach dem Äußeren der Vorratskammer zwischen der ersten (40) und der zweiten Öffnung (41) ausbilden, wobei eine Fluidverbindung der Pumpe (50) zu der Vorratskammer (35) ermöglicht wird.

22. Aufbau nach einem der voranstehenden zwei Ansprüche, **dadurch gekennzeichnet, dass** der Deckel (80) mit dem Körper (26), wenn diese verbunden sind, einen Durchgang zur Luftnachführung festlegt.

23. Aufbau nach irgendeinem der Ansprüche 1 bis 12, **dadurch gekennzeichnet, dass** die nachfüllbare Einheit (105) aufweist, einen Körper (120) und einen Kolben (153), der im Hinblick auf den Körper (25) bewegbar ist und mit diesem eine Vorratskammer mit einem veränderbaren Volumen (155) festlegt.

24. Aufbau nach dem voranstehenden Anspruch, **dadurch gekennzeichnet, dass**, wenn die nachfüllbare Einheit (105) auf dem Behälter (102) in der Weise angeordnet ist, dass die Pumpe Produkt in dem Behälter entnehmen kann, die Pumpe insbesondere mit einem Tauchrohr (17) des Behälters in Verbindung steht, wobei das Volumen der Vorratskammer (155) minimal ist.

25. Aufbau nach irgendeinem der zwei voranstehenden Ansprüche, **dadurch gekennzeichnet, dass** die Vorratskammer (155) unter dem Kolben (153) ausgebildet ist, wenn die nachfüllbare Einheit mit dem Kopf nach oben zu beobachten ist.

26. Aufbau nach dem voranstehenden Anspruch, **dadurch gekennzeichnet, dass** die nachfüllbare Einheit (105) aufweist, eine im Hinblick auf den Körper (120) bewegbare Anordnung (119), wobei die bewegbare Anordnung (119) den Kolben (153) umfasst.

27. Aufbau nach dem voranstehenden Anspruch, **dadurch gekennzeichnet, dass** die bewegbare Anordnung (119) aufweist, eine äußere Manschette (123), die auf dem Körper (120) geführt ist.

28. Aufbau nach einem der zwei voranstehenden Ansprüche, **dadurch gekennzeichnet, dass** die bewegbare Anordnung (119) eine Pumpe (150) aufweist.

29. Aufbau nach dem voranstehenden Anspruch, **dadurch gekennzeichnet, dass** die Pumpe (150) eine Leitung (151) aufweist, wobei der Kolben (153) um die Leitung herum festgelegt ist.

30. Aufbau nach dem voranstehenden Anspruch, **dadurch gekennzeichnet, dass** der Kolben (153) eine untere Fläche hinter dem unteren Ende der Leitung (151) vorzuweisen hat, wobei letztere einen Abschnitt (158) aufweist, der sich in Richtung des unteren Teils unter dem Kolben (153) erstreckt bzw. fortsetzt.

31. Aufbau nach irgendeinem der Ansprüche 25 bis 30, **dadurch gekennzeichnet, dass** der Körper (120) der nachfüllbaren Einheit (105) aufweist, eine öffnung (132), die es erlaubt, eine Fluidverbindung der Vorratskammer (155) mit dem Behälter (102) einzurichten.

32. Aufbau nach dem voranstehenden Anspruch, **dadurch gekennzeichnet, dass** die nachfüllbare Einheit (105) aufweist, einen Deckel (170), der geeignet ist, um in einer abnehmbaren Weise insbesondere durch Verschraubung oder Verrastung, auf dem Körper (120) festgelegt zu sein, um die Öffnung (132) zu verschließen, wenn die nachfüllbare Einheit (105) von dem Behälter (102) getrennt ist.

33. Aufbau nach irgendeinem der voranstehenden Ansprüche, **dadurch gekennzeichnet, dass** der Behälter ein kosmetisches Produkt inklusive zur Pflege, insbesondere ein Parfum, enthält.

34. Aufbau nach dem voranstehenden Anspruch, **dadurch gekennzeichnet, dass** das Produkt eine Creme ist.

35. Verfahren zum Verteilen eines Produktes, das nachfolgende Schritte aufweist:
- ein Aufbau (1; 101) zur Aufbereitung und zur Verteilung wird zusammengesetzt, der aufweist:
- einen Behälter (2; 102; 3'), um einen Vorrat des Produktes zu enthalten,
- eine nachfüllbare Einheit (3; 105), die angeordnet ist, um in einer abnehmbaren bzw. bewegbaren Weise auf dem Behälter angeordnet zu sein, und die eine Vorratskammer (35; 155) und eine Pumpe (50; 150) aufweist, **gekennzeichnet durch** die nachfolgenden Schritte:
- Entnehmen des Produktes mit Hilfe der nachfüllbaren Einheit entweder in dem Behälter, wenn die nachfüllbare Einheit auf dem Behälter angeordnet ist, oder in der Vorratskammer der nachfüllbaren Einheit, wenn die nachfüllbare Einheit von dem Behälter getrennt ist,
- Verteilen einer Menge des entnommenen Produkts mit Hilfe der nachfüllbaren Einheit, indem die Pumpe über einen vollständigen Hub betätigt wird, wobei die ausgelieferte Menge einen Bruchteil lediglich des maximalen Volumens der Vorratskammer entspricht, insbesondere wenigstens einem Zehntel.

36. Verfahren nach dem voranstehenden Anspruch, wobei die Pumpe eine Auslassöffnung (63) und der Behälter eine Ausgangsöffnung (21; 132) aufweist, **dadurch gekennzeichnet, dass** das Verfahren die nachfolgenden Schritte aufweist:
- die Auslassöffnung (63) wird in Verbindung mit der Ausgangsöffnung (21; 132) gebracht,
- das Produkt wird in dem Behälter entnommen.

37. Verfahren nach einem der voranstehenden zwei Ansprüche, **dadurch gekennzeichnet, dass** es nachfolgende Schritte aufweist:
- die nachfüllbare Einheit (3) wird auf dem Behälter (2) angeordnet, wobei die Anordnung mit dem Kopf nach unten ist,
- die Pumpe (50) wird wiederholte Male über einen vollständigen Ablauf betätigt, um einen Unterdruck in der Vorratskammer zu erzeugen, um die Befüllung bzw. Auffüllung von dieser mit Produkt zu erlauben, ohne dass das Produkt zwischen zwei vollständigen Abläufen verteilt werden sollte.

38. Verfahren nach Anspruch 36, wobei die nachfüllbare Einheit aufweist, einen Körper und einen Kolben, der an dem Körper geführt ist, **dadurch gekennzeichnet, dass** das Verfahren die nachfolgenden Schritte aufweist:
- die nachfüllbare Einheit (105) wird auf dem Behälter (102) angeordnet,
- der Kolben wird relativ zu dem Körper verbracht, um das Volumen der Vorratskammer zu vergrößern, wobei der Kolben an der Pumpe anlässlich der Verbringung festgelegt ist.
